# EUROPÄISCHE PATENTANMELDUNG

(11) **EP 0 598 348 A1**
(43) Veröffentlichungstag der Anmeldung: **25.05.1994**
(21) Anmeldenummer: 93118310.7
(22) Anmeldetag: 11.11.1993
(51) Int. Cl.: A61B 17/39

(54) **Bipolares Hochfrequenzschneidinstrument**

(30) Priorität: 16.11.1992 DE 9215589 U
(71) Anmelder: DELMA ELEKTRO-UND MEDIZINISCHE APPARATEBAU GESELLSCHAFT mbH, D-78532 Tuttlingen (DE)
(72) Erfinder: Fritzsch, Gernod, D-78532 Tuttlingen (DE)
(74) Vertreter: Morgan, James G.

(57) **Zusammenfassung**

Ein bipolares Hochfrequenzschneidinstrument weist einen Schaft (11) und zwei darin vorhandene Elektrodenträger (12, 13) auf, welche aufeinander zu bzw. voneinander weg bewegbar sind und an ihrem aus dem Schaft (11) heraustretenden Ende zwei Elektroden (14, 15) in Form eines Schneidedrahtes (18) und einer damit zusammenarbeitenden Gabel (19) aufweist.

## Beschreibung

Die Erfindung betrifft ein bipolares Hochfrequenzschneidinstrument nach dem Oberbegriff des Anspruches 1.

Um die Patientenbelastung zu senken und die Liegedauern in den Krankenhäusern zu verkürzen, werden zunehmend Operationen durchgeführt, bei denen Spezialinstrumente unter Sichtkontrolle mittels Endoskopen durch sog. Trokare in den Patientenkörper eingeführt werden. Für derartige Operationen werden besondere Hochfrequenzschneidgeräte benötigt, die neben den Endoskopen durch die Trokare in das Körperinnere eingeführt werden können.

Ein für diese Zwecke geeignetes kombiniertes Hochfrequenz-Koagulations-Schneidinstrument ist bereits vorgeschlagen worden (DE-OS 41 38 116).

Das Ziel der vorliegenden Erfindung besteht darin, ein bipolares Hochfrequenzschneidinstrument zu schaffen, das in besonderem Maße für das Hochfrequenzschneiden bei der endoskopischen Chirurgie geeignet ist.

Zur Lösung dieser Aufgabe sind die Merkmale des kennzeichnenden Teils des Anspruches 1 vorgesehen. Vorteilhafte Weiterbildungen der Erfindung sind durch die Unteransprüche gekennzeichnet.

Aufgrund dieser Ausbildung kann der Schaft des Schneidinstrumentes bei in der Schneidstellung befindlichen Schneidelektroden durch das Trokar zum Operationsfeld geführt werden, worauf dann durch axiale Relativverschiebung zwischen dem Schaft und den Elektrodenträgern die Elektroden in die Erfassungsstellung gebracht werden können. Durch anschließende erneute Relativverschiebung zwischen dem Schaft und den Elektrodenträgern kann dann nach Einschalten der Hochfrequenz ein Schneidvorgang durchgeführt werden.

Es können sowohl der Schaft als auch die Elektrodenträger axial verschoben werden, um die Elektroden zu betätigen.

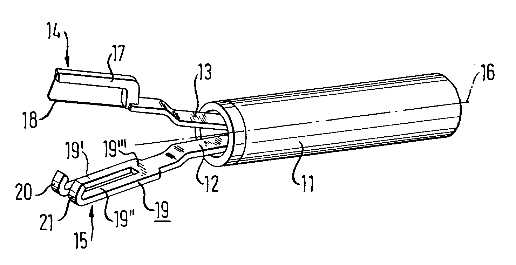

Die Erfindung wird im folgenden beispielsweise anhand der Zeichnung beschrieben; in dieser zeigt die einzige Figur eine perspektivische Ansicht des vorderen Endes eines erfindungsgemäßen Hochfrequenzschneidinstrumentes.

Nach der Zeichnung ragen aus dem vorderen Ende eines vorzugsweise aus Metall bestehenden rohrförmigen Schaftes 11, der innen und außen isoliert sein kann, stabförmige Elektrodenträger 12, 13 heraus, an denen eine Schneidelektrode 14 bzw. eine Gegenelektrode 15 befestigt sind. Durch axiale Relativverschiebung zwischen dem rohrförmigen Schaft 11 einerseits und den Elektrodenträgern 12, 13 andererseits können die Elektroden 14, 15 voneinander weg (siehe Zeichnung) oder aufeinander zu in eine Schneidstellung bewegt werden. Der Mechanismus für diese Betätigung der Elektroden ist der gleiche, wie er in der DE-PS 27 34 847 beschrieben ist. Für diese Art der Elektrodenbetätigung ist es wichtig, daß die Elektrodenträger 12, 13, wenn sie sich in der aus der Zeichnung ersichtlichen Erfassungsstellung befinden, derart federnde Eigenschaften aufweisen, daß sie sich selbsttätig federnd voneinander wegspreizen. Gleichzeitig dienen die Elektrodenträger 12, 13 als Stromzuführung für die Elektroden 14, 15, haben also eine Doppelfunktion.

Die in der Zeichnung obere Schneidelektrode 14 besteht aus einem L-förmigen Halter 17 aus Metall, an dem komplementär ein L-förmig gebogener Schneidedraht 18 befestigt ist. Die gegenüberliegende Gegenelektrode 15 ist als metallische Gabel 19 ausgebildet, deren Steg 19''' elektrisch und mechanisch mit dem Elektrodenträger 12 verbunden ist. Von dem Steg 19''' erstrecken sich die beiden Schenkel 19', 19'' der Gabel 19, welche insgesamt flach, d.h. eben ausgebildet ist. Am vorderen Ende der Schenkel 19', 19'' sind Abwinklungen 20 bzw. 21 vorgesehen, die sich in Richtung des Schneidedrahtes 18 erstrecken und der hakenförmigen Erfassung von Gewebe dienen.

Der geradlinige Teil des Schneidedrahtes 18, der die eigentliche Schneidefunktion ausführt, taucht bei durch Vorschieben des Schaftes 11 aufeinanderzu bewegten Elektroden 14, 15 in den Schlitz zwischen den Schenkeln 19', 19'' der Gabel 19 zumindest so tief ein, daß dabei vom Schneidedraht 18 erfaßtes und gegen die Schenkel 19', 19'' abgestütztes Gewebe oder dergl. durch Hochfrequenzschneiden vollständig abgetrennt wird.

Das zu schneidende Gewebe kann vorher durch die hakenförmigen Abwinklungen 20, 21 erfaßt und so beim anschließenden Schneidvorgang sicher gehalten werden.

Der Schneidedraht 18 kann nicht nur die aus der Figur ersichtliche L-Form haben, sondern beispielsweise auch U- oder trapezförmig ausgebildet und an einem sich in Richtung der Längsachse 16 des Schaftes 11 erstreckenden Metallhalter 17 in geeigneter Weise befestigt sein.

In jedem Fall ist der Halter 17 von der Gabel 19 abgewandt, während der die Schneidfunktion ausübende Schneidedraht 18 zumindest mit seinem schneidenden geraden Teil, der zwischen die Schenkel 19', 19'' eintritt, der Gabel 19 zugewandt ist.

## Patentansprüche

1. Bipolares Hochfrequenzschneidinstrument, insbesondere zur Durchführung von endoskopischen Operationen, mit einem rohrförmigen Schaft (11) und zwei in dessen Innerem in seiner Längsrichtung angeordneten, voneinander isolierten stabförmigen Elektrodenträgern (12, 13), deren aus dem vorderen Ende des Schaftes (11) herausragende Enden zwei miteinander zusammenwirkende Elektroden (14, 15), und zwar eine Schneidelektrode (14) und eine Gegenelektrode (15) tragen und durch eine axiale Bewegung relativ zum Schaft (11) in der einen oder anderen Richtung wahlweise aufeinander zu und voneinander weg bewegbar sind, derart, daß die Elektroden (14, 15) aus einer Erfassungsstellung, wo sie einen deutlichen Abstand voneinander aufweisen, in eine Schneidstellung, wo sie Gewebe oder dergl. zerschnitten haben, und umgekehrt im wesentlichen quer zur Längsrichtung (16) des Schaftes (11) verschiebbar sind, wobei die Elektroden (14, 15) gegebenenfalls über einen im Inneren des Schaftes (11) axial verschiebbar angeordneten stabförmigen Kontaktträger an ein Hochfrequenz-Chirurgiegerät anschließbar sind, welches die Elektroden (14, 15) vorzugsweise über die Elektrodenträger (12, 13) mit einem für das Schneiden geeigneten Hochfrequenzstrom versorgt,
dadurch gekennzeichnet,
daß die eine Schneidelektrode (14) als Halter (17) mit daran angeordnetem Schneidedraht (18) ausgebildet ist und die Gegenelektrode (15) eine Gabel (19) ist, zwischen deren Schenkeln (19', 19'') einerseits und dem darin eingeführten Schneidedraht (18) andererseits der Hochfrequenz-Schneidvorgang stattfindet.

2. Hochfrequenzschneidinstrument nach Anspruch 1,
dadurch gekennzeichnet,
daß die beiden Elektroden (14, 15) im geöffneten Zustand eine V-förmige Öffnung bilden, die einen Öffnungswinkel zwischen 10° und 75°, vorzugsweise zwischen 15° und 35° aufweist.

3. Hochfrequenzschneidinstrument nach Anspruch 1 oder 2,
dadurch gekennzeichnet,
daß in der Schneidstellung der Schneidedraht (18) in den Zwischenraum zwischen den Schenkeln (19', 19'') der Gabel eingetaucht ist.

4. Hochfrequenzschneidinstrument nach einem der vorhergehenden Ansprüche,
dadurch gekennzeichnet,
daß der Schneidedraht (18) entweder ganz oder teilweise im wesentlichen geradlinig ausgebildet ist und in der Schneidstellung im wesentlichen parallel zur Längsachse (16) des Schaftes (11) verläuft, und zwar entweder zwischen den Schenkeln (19', 19'') oder etwas darunter.

5. Hochfrequenzschneidinstrument nach einem der vorhergehenden Ansprüche,
dadurch gekennzeichnet,
daß die Schenkel (19', 19'') der Gabel (19) geradlinig sind und in der Schneidstellung im wesentlichen parallel zur Längsachse (16) des Schaftes (11) verlaufen.

6. Hochfrequenzschneidinstrument nach einem der vorhergehenden Ansprüche,
dadurch gekennzeichnet,
daß die Schenkel (19', 19'') der Gabel (19) am vorderen Ende mit Abwinklungen (20, 21) versehen sind.

7. Hochfrequenzschneidinstrument nach Anspruch 6,
dadurch gekennzeichnet,
daß die Abwinklungen (20, 21) aus der Ebene der vorzugsweise flach ausgebildeten Gabel (19) herausgebogen sind.

8. Hochfrequenzschneidinstrument nach Anspruch 6 oder 7,
dadurch gekennzeichnet,
daß die Abwinklungen (20, 21) um etwas mehr als 90° aus der Ebene der Gabel (19) herausgebogen sind, derart, daß hakenförmige Greifer am vorderen Ende der Gabel (19) vorliegen.

9. Hochfrequenzschneidinstrument nach einem der vorhergehenden Ansprüche,
dadurch gekennzeichnet,
daß der Halter (17) L-Form aufweist, wobei der kurze Schenkel oben angeordnet und mit dem Elektrodenträger (13) verbunden ist und der lange Schenkel an seinem vorderen Ende den ebenfalls L-förmig ausgebildeten Schneidedraht (18) trägt, dessen kurzer Schenkel sich am vorderen Ende befindet und dessen langer Schenkel unten angeordnet ist und in der Schneidestellung parallel zu den Gabelschenkeln (19', 19'') verläuft.

10. Hochfrequenzschneidinstrument nach einem der Ansprüche 1-8,
dadurch gekennzeichnet,
daß der Halter (17) U-förmig ausgebildet ist und der Schneidedraht (18) gegenüber dem oben befindlichen U-Steg unten geradlinig zwischen den Enden der U-Schenkel angeordnet ist.
